(19) Europäisches Patentamt
European Patent Office
Office européen des brevets

(11) EP 2 249 880 B1

(12) **EUROPÄISCHE PATENTSCHRIFT**

(45) Veröffentlichungstag und Bekanntmachung des Hinweises auf die Patenterteilung:
**29.03.2017 Patentblatt 2017/13**

(21) Anmeldenummer: **09716918.9**

(22) Anmeldetag: **27.02.2009**

(51) Int Cl.:
***A61L 15/46*** *(2006.01)* ***A61L 15/60*** *(2006.01)*
***C08K 5/098*** *(2006.01)* ***A61K 31/60*** *(2006.01)*

(86) Internationale Anmeldenummer:
**PCT/EP2009/052347**

(87) Internationale Veröffentlichungsnummer:
**WO 2009/109524 (11.09.2009 Gazette 2009/37)**

(54) **SUPERABSORBIERENDE ZUSAMMENSETZUNG MIT METALLSALICYLAT ZUR GERUCHSKONTROLLE**

SUPERABSORBENT COMPOSITION WITH METAL SALICYLATE FOR ODOUR CONTROL

COMPOSITION SUPERABSORBANTE AVEC SALICYLATE MÉTALLIQUE POUR L ÉLIMINATION DES ODEURS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **05.03.2008 DE 102008012728**

(43) Veröffentlichungstag der Anmeldung:
**17.11.2010 Patentblatt 2010/46**

(73) Patentinhaber: **Evonik Degussa GmbH**
**45128 Essen (DE)**

(72) Erfinder:
- **Wattebled, Laurent**
**40589 Düsseldorf (DE)**
- **Furno, Franck**
**40545 Düsseldorf (DE)**

(56) Entgegenhaltungen:
**EP-A- 0 889 063**
**WO-A-01/70210**
**WO-A-03/028778**
**WO-A-2007/057203**
**WO-A-2007/122343**
**US-A1- 2006 029 567**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 249 880 B1

## Beschreibung

Die vorliegende Erfindung betrifft eine wasserabsorbierende Zusammensetzung, ein Verfahren zur Herstellung einer wasserabsorbierenden Zusammensetzung, die durch dieses Verfahren erhältliche, wasserabsorbierende Zusammensetzung, einen Verbund, ein Verfahren zur Herstellung eines Verbundes, den durch dieses Verfahren erhältlichen Verbund, chemische Produkte wie beispielsweise Hygieneartikel, die Verwendung einer wasserabsorbierenden Zusammensetzung oder eines Verbundes sowie die Verwendung einer definierten Verbindung.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter Druck zurückzuhalten. Im Allgemeinen betragen diese Flüssigkeitsaufnahmen das mindestens 10-Fache oder gar das mindestens 100-Fache des Trockengewichts der Superabsorber bzw. der superabsorbierenden Zusammensetzungen an Wasser. Durch diese charakteristischen Eigenschaften finden diese Polymere hauptsächlich Anwendung in Sanitärartikeln wie Babywindeln, Inkontinenzprodukten oder Damenbinden. Einen umfassenden Überblick über Superabsorber bzw. superabsorbierende Zusammensetzungen, ihre Anwendung und ihre Herstellung geben F. L. Buchholz und A. T. Graham (Herausgeber) in "Modern Superabsorbent Polymer Technology", Wiley-VCH, New York, 1998.

Die Herstellung der Superabsorber erfolgt in der Regel durch die radikalische Polymerisation säuregruppentragender, meist teilneutralisierter Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

DE 40 20 780 C1 offenbart die Nachbehandlung superabsorbierender Polymere durch Nachvernetzung der Oberflächen der Polymerteilchen. Durch die Nachvernetzung der Oberfläche der wasserabsorbierenden Polymerteilchen wird insbesondere das Absorptionsvermögen der Polymerteilchen unter der Einwirkung von Drücken erhöht.

DE 199 09 653 A1 und DE 199 09 838 A1 beschreiben pulverförmige, an der Oberfläche nachvernetzte, Wasser, wässrige oder seröse Flüssigkeiten oder Blut absorbierende Polymerisate, welche auf säuregruppen-tragenden Monomeren basieren und welche mit einem Oberflächennachvernetzungsmittel und einem Kation in wässriger Lösung beschichtet und nachvernetzt worden sind. Die in diesem Stand der Technik offenbarten Polymerisate weisen gegenüber herkömmlichen Polymerisaten vorteilhafte Absorptionseigenschaften, insbesondere eine hohe Permeabilität auf.

Beim längeren Tragen von absorbierende Polymere beinhaltenden Hygieneartikeln, welche bereits zu einem Teil Körperflüssigkeiten wie Urin aufgenommen haben, oder aber beim Lagern von benutzten Windeln, wie es beispielsweise in Krankenhäusern üblich ist, kann es bedingt durch die organischen Bestandteile des Urins und die Körperwärme der Trägerperson alsbald zu einer unangenehmen Geruchsbelastung kommen. Um dieser zu begegnen, wurden zahlreiche Versuche unternommen, durch entsprechende Beigaben in den vom Superabsorber verschiedenen Bestandteilen des Hygieneartikels eine Bindung der geruchsbildenden Stoffe zu erzielen oder den Geruch durch Parfums oder dergleichen zu übertönen. Das Einbringen von derartigen Substanzen in der Form von von Superabsorbern verschiedenen Bestandteilen wirkt sich oftmals negativ auf die Performance dieser Hygieneartikel während des Tragens aus. So werden durch die Körperflüssigkeiten oftmals die anfänglich vom Superabsorberbereich räumlich getrennt vorliegenden geruchshemmenden oder geruchsvermindernden Substanzen beispielsweise durch Hineinschwemmen in Superabsorber beinhaltende Bereich eines Hygieneartikels eingetragen, wo sie dann eine negative Auswirkung auf die Performance des Superabsorbers und damit des Hygieneartikels insgesamt zeigen. Ferner ist an diesem Konzept nachteilig, dass der Großteil der in den Hygieneartikel abgegebenen Körperflüssigkeit sich ohnehin in dem Superabsorber befindet und daher die außerhalb des Superabsorbers befindlichen geruchshemmenden oder geruchsvermindernden Substanzen ihre Wirkung schlechter entfalten können.

DE 198 25 486 A1 und DE 199 39 662 A1 offenbaren die Kombination von Superabsorbern mit Cyclodextrin zur Geruchsverminderung. Diesem durchaus viel versprechenden Ansatz ist jedoch zu entnehmen, dass das Cyclodextrin nur unter bestimmten Bedingungen, nämlich wenn sichergestellt ist, dass das Cyclodextrin nicht von dem Superabsorber wieder separiert, seine geruchshemmende Wirkung in dem Superabsorber zeigt. Hierbei ist es bevorzugt, dass das Cyclodextrin zumindest in die Oberfläche des Superabsorberartikels eingearbeitet ist, indem Cyclodextrin und/oder Cyclodextrinderivate kovalent und/oder ionisch gebunden und/oder darin eingeschlossen sind.

Aus DE 103 34 271 A1 sind weiterhin Superabsorberagglomerate bekannt, die eine Vielzahl von Geruchsbindern homogen in dem Agglomerat aufweisen können. Diese eine hervorragende Lösung für die Verwendung von Superabsorberfeinteilchen offenbarende Schrift stellt jedoch keine für die Anwendung in Hygieneartikeln besonders gut geeignete Superabsorber mit Geruchsbindungseigenschaften zur Verfügung. So sind neben einem effizienten und wirkungsvollen Einsatz der Geruchsbinder auch die durch diesen Geruchsbinder beeinflussten Superabsorbereigenschaften noch verbesserungswürdig.

DE 10 2005 055 497 A1 lehrt, superabsorbierende Polymere dadurch das in Kontakt bringen mit Metallsalzen

der Ricinolsäure, gegebenenfalls in Kombination mit Aminosäuren zur Löslichkeitsverbesserung, mit verbesserten geruchsbindenden Eigenschaften zu versehen.

Im Allgemeinen lag der vorliegenden Erfindung die Aufgabe zugrunde, die sich aus dem Stand der Technik ergebenden Nachteile abzumildern oder gar zu überwinden.

Eine erfindungsgemäße Aufgabe bestand insbesondere darin, eine wasserabsorbierende Zusammensetzung beinhaltend einen Superabsorber und ein geruchsbindendes Additiv bereitzustellen, die über gute Geruchsbindungseigenschaften verfügt. Neben der vorteilhaften Geruchsbindungseigenschaft soll sich die wasserabsorbierende Zusammensetzung auch durch eine gute Performance in einem Hygieneartikel auszeichnen. Insbesondere sollte die Performance eines Hygieneartikels, der diese wasserabsorbierende Zusammensetzung beinhaltet, im Wesentlichen gleich gut oder gar besser sein als die Performance des Hygieneartikels mit einem Superabsorber, der das geruchsbindende Additiv nicht beinhaltet.

Weiterhin sollte insbesondere die Rieselfähigkeit der wasserabsorbierenden Zusammensetzung durch die Verwendung des geruchsbindenden Additivs möglichst gar nicht oder allenfalls geringfügig beeinflusst werden, so dass sich die wasserabsorbierende Zusammensetzung im Vergleich zu den aus dem Stand der Technik bekannten, ein geruchsbindendes Additiv beinhaltenden Zusammensetzungen einfacher fördern und prozessieren lässt. Darüber hinaus sollte die Permeabilität der wasserabsorbierenden Zusammensetzung für wässrige Flüssigkeiten im Vergleich zu den aus dem Stand der Technik bekannten geruchsbindenden wasserabsorbierenden Zusammensetzungen mindestens vergleichbar, vorteilhafterweise jedoch weiter verbessert sein.

Auch sollte die Menge, in der das geruchsbindende Additiv in der wasserabsorbierenden Zusammensetzung enthalten ist, im Vergleich zu den aus dem Stand der Technik bekannten geruchsbindenden wasserabsorbierenden Zusammensetzungen bei gleicher Geruchsbindungskapazität möglichst vermindert sein.

Ferner bestand eine erfindungsgemäße Aufgabe darin, ein Verfahren aufzuzeigen, mit dem eine solche wasserabsorbierende Zusammensetzung erhalten werden kann.

Zudem bestand eine erfindungsgemäße Aufgabe darin, einen Hygieneartikel bereitzustellen, der neben guten geruchsbindenden Eigenschaften auch eine gute Performance zeigt.

Außerdem lag der vorliegenden Erfindung als Aufgabe zugrunde, wasserabsorbierende Zusammensetzungen bereitzustellen, die sich allgemein in Verbunde einarbeiten lassen oder auch als Verbund oder als solche Verwendung in chemischen Produkten oder deren Bestandteilen finden können.

Einen Beitrag zur Lösung der eingangs genannten Aufgaben leistet eine wasserabsorbierende Zusammensetzung, beinhaltend wasserabsorbierende Polymergebilde , wobei die wasserabsorbierenden Polymergebilde einen Innenbereich und einen den Innenbereich umgebenden Aussenbereich aufweisen, wobei der Aussenbereich einen höheren Vernetzungsgrad aufweist als der Innenbereich, sowie eine Verbindung der Struktur I

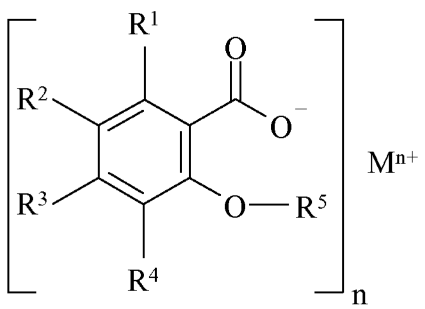

Struktur I

in der

- $R^1$, $R^2$, $R^3$ und $R^4$ gleich oder verschieden sein können und jeweils ein Wasserstoffatom, ein Halogen-Atom, beispielsweise ein Chlor-Atom oder ein Brom-Atom, eine $C_1$- bis $C_4$-Kohlenwasserstoff-Gruppe, vorzugsweise eine Methyl-Gruppe oder eine Ethyl-Gruppe, besonders bevorzugt eine Methyl-Gruppe, oder eine Hydroxyl-Gruppe,

- $R^5$ ein Wasserstoffatom, eine $C_1$- bis $C_4$-Kohlenwasserstoff-Gruppe oder eine Acetyl-Gruppe, vorzugsweise ein Wasserstoffatom oder eine Acetyl-Gruppe und am meisten bevorzugt ein Wasserstoffatom,

- n eine ganze Zahl ausgewählt aus der Gruppe bestehend aus 1, 2 oder 3, besonders bevorzugt die Zahl 1 oder die Zahl 2 und am meisten bevorzugt die Zahl 2,
  und

- $M^{n+}$ ein n-wertiges Metall-Kation oder ein $H^+$-Kation, vorzugsweise ein $H^+$-Kation, ein einwertiges Metall-Kation eines Alkalimetalls oder ein zweiwertiges Metall-Kation eines Erdalkali-Metalls oder eines Übergangsmetalls, besonders bevorzugt ein $H^+$-Kation, ein $Na^+$-Kation oder ein zweiwertiges Metall-Kation eines Übergangsmetalls der Gruppen 10, 11 oder 12, besonders bevorzugt der Gruppe 12 und am meisten bevorzugt ein $Zn^{2+}$-Kation

darstellen.

Gemäß einer besonders bevorzugten Ausführungsform der erfindungsgemäßen wasserabsorbierenden Zusammensetzung beinhaltet diese eine Verbindung der Struktur I, in der $R^1$, $R^2$, $R^3$, $R^4$ und $R^5$ ein Wasserstoffatom darstellen. Gemäß dieser besonderen Ausführungsform der erfindungsgemäßen Zusammensetzung handelt es sich bei der Verbindung der Struktur I um Salicylsäure (n = 1, $M^+ = H^+$), um Natriumsalicylat (n = 1, $M^+ = Na^+$) oder um Zinksalicylat (n = 2, $M^{2+} = Zn^{2+}$), wobei Zinksalicylat als Verbindung der Struktur I am meisten bevorzugt ist.

Auch ist es erfindungsgemäß bevorzugt, wenn die wasserabsorbierende Zusammensetzung die Verbindung der Struktur I in einer Menge in einem Bereich von 0,001 bis 10 Gew.-%, besonders bevorzugt in einem Bereich von 0,05 bis 5 Gew.-% und am meisten bevorzugt in einer Menge in einem Bereich von 0,1 bis 3 Gew.-%, jeweils bezogen auf das Gesamtgewicht der wasserabsorbierenden Polymergebilde, beinhaltet.

Weiterhin ist es erfindungsgemäß bevorzugt, dass die Verbindung der Struktur I in Form eines Pulvers und mithin in Form kleiner Partikel in der erfindungsgemäßen wasserabsorbierenden Zusammensetzung enthalten ist, wobei es weiterhin bevorzugt ist, dass ein solches Pulver höchstens 25 Gew.-%, noch mehr bevorzugt höchstens 10 Gew.-%, darüber hinaus bevorzugt höchstens 5 Gew.-%, jeweils bezogen auf das Gesamtgewicht der Verbindung der Struktur I, am meisten bevorzugt jedoch keine vorzugsweise vereinzelten Partikel beinhaltet, die größer als 700 μm, besonders bevorzugt größer als 500 μm und am meisten bevorzugt grö-ßer als 300 μm im Durchmesser sind. Jedoch können in dem Pulver der Verbindung I durchaus auch Agglomerate aus mindestens zwei Partikeln enthalten sein, die größer als 700 μm, 500 μm bzw. 300 μm sind, wobei die Menge dieser Agglomerate durchaus auch größer als 25 Gew.-%, 10 Gew.-% bzw. 5 Gew.-% sein kann. Insbesondere kann es sich als vorteilhaft erweisen, dass das Pulver der Verbindung der Struktur I zu mindestens 50 Gew.-%, noch mehr bevorzugt zu mindestens 90 Gew.-% und am meisten bevorzugt ausschließlich auf Partikeln mit einem Durchmesser in einem Bereich von 50 bis 800 μm, besonders bevorzugt 50 bis 500 μm und am meisten bevorzugt 50 bis 350 μm basiert.

Weiterhin ist es erfindungsgemäß bevorzugt, wenn die Pulver-Partikel der Verbindung der Struktur I über einen Binder auf der Oberfläche der wasserabsorbierenden Polymergebilde immobilisiert sind, wobei als Binder grundsätzlich organische oder anorganische Binder eingesetzt werden können. Als Binder kommen dabei insbesondere thermoplastische Schmelzklebstoffe, hydrophile, vorzugsweise wasserlösliche Polymere oder auch Wasser selbst in Betracht. Als thermoplastische Schmelzklebstoffe können dabei insbesondere diejenigen partikulären Verbindungen eingesetzt werden, die in der WO-A-2005/011860 als thermoplastische Klebstoffe genannt sind. Als hydrophile, vorzugsweise wasserlösliche Polymere können insbesondere Polyvinylalkohole, die durch teilweises oder vollständiges Verseifen von Polyvinylacetat erhalten werden können, oder Polyalkylenglykole, insbesondere Polyethylenglykole mit einem Molekulargewicht in einem Bereich von 1.000 bis 50.000 g/mol, besonders bevorzugt in einem Bereich von 1.500 bis 25.000 g/mol und am meisten bevorzugt in einem Bereich von 2.000 bis 15.000 g/mol eingesetzt werden.

Die in der erfindungsgemäßen Zusammensetzung enthaltenen, wasserabsorbierenden Polymergebilde basieren vorzugsweise auf teilneutralisierten, vernetzten Polyacrylaten.

Dabei sind erfindungsgemäß bevorzugte wasserabsorbierende Polymergebilde insbesondere Fasern, Schäume oder Teilchen, wobei Fasern und Teilchen besonders bevorzugt und Teilchen am meisten bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 μm, vorzugsweise 20 bis 2000 μm und besonders bevorzugt 150 bis 850 μm oder 150 bis 600 μm aufweisen. Dabei ist es insbesondere bevorzugt, dass der Anteil der Polymerteilchen mit einer Partikelgröße in einem Bereich von 300 bis 600 μm mindestens 30 Gew.-%, besonders bevorzugt mindestens 40 Gew.-%, darüber hinaus bevorzugt mindestens 50 Gew.-% und am meisten bevorzugt mindestens 75 Gew.-%, bezogen auf das Gesamtgewicht der wasserabsorbierenden Polymerteilchen, beträgt. Gemäß einer anderen Ausführungsform der erfindungsgemäßen wasserabsorbierenden Polymergebilde beträgt der Anteil der Polymerteilchen mit einer Partikelgröße in einem Bereich von 150 bis 850 μm mindestens 50 Gew.-%, besonders bevorzugt mindestens 75 Gew.-%, darüber hinaus bevorzugt mindestens 90 Gew.-% und am meisten bevorzugt mindestens 95 Gew.-%, bezogen auf das Gesamtgewicht der wasserabsorbierenden Polymerteilchen.

In einer bevorzugten Ausführungsform der in der erfindungsgemäßen wasserabsorbierenden Zusammensetzung enthaltenen wasserabsorbierenden Polymergebilde basieren diese auf

($\alpha$1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,

($\alpha$2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,

($\alpha$3) 0,001-5 Gew.-%, vorzugsweise 0,01-3 Gew.-% und besonders bevorzugt 0,01-2,5 Gew.-% eines oder mehrerer Vernetzer,

($\alpha$4) 0-30 Gew.-%, vorzugsweise 0-5 Gew.-% und besonders bevorzugt 0,1-5 Gew.-% eines wasserlöslichen Polymeren,

($\alpha$5) 0-20 Gew.-%, vorzugsweise 2,5-15 Gew.-% und besonders bevorzugt 5-10 Gew.-% Wasser, sowie

($\alpha$6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen ($\alpha$1) bis ($\alpha$6) 100 Gew.-% beträgt.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. Gemäß besondere Ausführungsformen der erfindungsgemäßen Zusammensetzung beinhaltet dieses jedoch wasserabsorbierende Polymergebilde, welche zu höchstens 78 Mol-%, besonders bevorzugt zu höchstens 70 Mol-% und zu mindestens 50 Mol-% neutralisiert sind. In diesem Zusammenhang kann gemäß einer Ausgestaltung der erfindungsgemäßen Zusammensetzung der Neutralisationsgrad der wasserabsorbierenden Polymergebilde in einem Bereich von 65 bis 80 Mol-%, besonders bevorzugt in einem Bereich von 70 bis 78 Mol-% und gemäß einer anderen Ausgestaltung in einem Bereich von 45 bis 65 Mol-%, besonders bevorzugt in einem Bereich von 50 bis 60 Mol-% liegen. Weiterhin wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhält. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere ($\alpha$1) sind vorzugsweise diejenigen Verbindungen, die in der WO 2004/037903 A2, die hiermit als Referenz eingeführt wird und somit als Teil der Offenbarung gilt, als ethylenisch ungesättigte, säuregruppenhaltige Monomere ($\alpha$1) genannt werden. Besonders bevorzugte ethylenisch ungesättigte, säuregruppenhaltige Monomere ($\alpha$1) sind Acrylsäure und Methacrylsäure, wobei Acrylsäure am meisten bevorzugt ist.

Gemäß einer Ausführungsform des erfindungsgemäßen Verfahrens werden wasserabsorbierende Polymergebilde eingesetzt, bei denen die monoethylenisch ungesättigten, mit ($\alpha$1) copolymerisierbare Monomere ($\alpha$2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Auch kann es sich bei den monoethylenisch ungesättigten, mit (α1) copolymerisierbaren Monomeren (α2) um wasserlösliche Monomere handeln. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat.

Die monoethylenisch ungesättigten, mit (α1) copolymerisierbaren Monomere (α2) umfassen weiterhin Methylpolyethylenglykolallylether, Vinylacetat, Styrol und Isobutylen.

Als Vernetzer (α3) werden vorzugsweise diejenigen Verbindungen eingesetzt, die in der WO 2004/037903 A2 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den wasserabsorbierenden Polymergebilden wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel (α6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der in der erfindungsgemäßen wasserabsorbierenden Zusammensetzung enthaltenen wasserabsorbierenden Polymergebilde basieren diese zu mindestens 50 Gew.-%, vorzugsweise zu mindestens 70 Gew.-% und darüber hinaus bevorzugt zu mindestens 90 Gew.-% auf carboxylatgruppen-tragenden Monomeren. Es ist erfindungsgemäß weiterhin bevorzugt, dass die Komponente (α1) zu mindestens 50 Gew.-% neutralisiert ist, wobei gemäß einer Ausgestaltung des erfindungsgemäßen Verfahrens der Neutralisationsgrad der wasserabsorbierenden Polymergebilde in einem Bereich von 65 bis 80 Mol-%, besonders bevorzugt in einem Bereich von 70 bis 78 Mol-% und gemäß einer anderen Ausgestaltung in einem Bereich von 45 bis 65 Mol-%, besonders bevorzugt in einem Bereich von 50 bis 60 Mol-% liegen kann.

Weiterhin weisen die wasserabsorbierenden Polymergebilde einen Innenbereich und einen den Innenbereich umgebenden Aussenbereich auf, wobei der Aussenbereich einen höheren Vernetzungsgrad aufweist als der Innenbereich. Eine solcher inhomogener Vernetzungsgrad lässt sich dadurch erzielen, dass die wasserabsorbierenden Polymergebilde durch die Beschichtung mit einem geeigneten Oberflächennachvernetzer, welcher mindestens zwei funktionelle Gruppen aufweist, die mit den funktionellen Gruppen im Aussenbereich des wasserabsorbierenden Polymergebildes reagieren können, nachvernetzt wird.

Weiterhin ist es erfindungsgemäß bevorzugt, dass die wasserabsorbierende Zusammensetzung erhältlich ist durch das in Kontakt bringen der wasserabsorbierender Polymergebilde mit der Verbindung der Struktur I, wobei es insbesondere bevorzugt ist, dass die Verbindung der Struktur I in einer Menge in einem Bereich von 0,01 bis 10 Gew.-%, besonders bevorzugt in einer Menge in einem Bereich von 0,05 bis 5 Gew.-% und am meisten bevorzugt in einer Menge in einem Bereich von 0,1 bis 3 Gew.-%, jeweils bezogen auf das Gesamtgewicht der wasserabsorbierenden Polymergebilde, mit den wasserabsorbierenden Polymergebilden in Kontakt gebracht wird. Das in Kontakt bringen erfolgt dabei vorzugsweise durch einfaches Vermischen der Verbindung der Struktur I mit den wasserabsorbierenden Polymergebilden, gegebenenfalls in Gegenwart des vorstehend genannten Binders.

Einen Beitrag zur Lösung der eingangs genannten Aufgaben leistet auch ein Verfahren zur Herstellung einer wasserabsorbierenden Zusammensetzung, beinhaltend die Verfahrensschritte:

i) Bereitstellen eines wasserabsorbierenden Polymergebildes;

ii) Nachvernetzung des wasserabsorbierenden Polymergebildes;

iii) in Kontakt bringen des wasserabsorbierenden Polymergebildes mit einer Verbindung der Struktur I

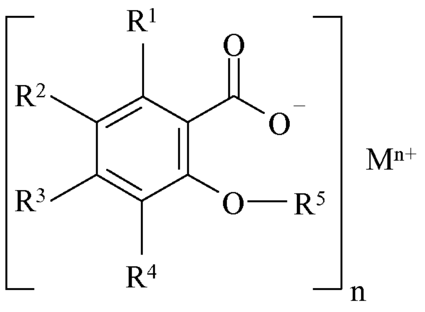

Struktur I

in der

- $R^1$, $R^2$, $R^3$ und $R^4$ gleich oder verschieden sein können und jeweils ein Wasserstoffatom, ein Halogen-Atom, beispielsweise ein Chlor-Atom oder ein Brom-Atom, eine $C_1$- bis $C_4$-Kohlenwasserstoff-Gruppe, vorzugsweise eine Methyl-Gruppe oder eine Ethylgruppe, besonders bevorzugt eine Methyl-Gruppe, oder eine Hydroxyl-Gruppe,
- $R^5$ ein Wasserstoffatom, eine $C_1$- bis $C_4$-Kohlenwasserstoff-Gruppe oder eine Acetyl-Gruppe, vorzugsweise ein Wasserstoffatom oder eine Acetyl-Gruppe und am meisten bevorzugt ein Wasserstoffatom,
- n eine ganze Zahl ausgewählt aus der Gruppe bestehend aus 1, 2 oder 3, besonders bevorzugt die Zahl 1 oder die Zahl 2 und am meisten bevorzugt die Zahl 2,

und

- $M^{n+}$ ein n-wertiges Metall-Kation oder ein $H^+$-Kation, vorzugsweise ein $H^+$-Kation, ein einwertiges Metall-Kation eines Alkalimetalls oder ein zweiwertiges Metall-Kation eines Erdalkali-Metalls oder eines Übergangsmetalls, besonders bevorzugt ein $H^+$-Kation, ein $Na^+$-Kation oder ein zweiwertiges Metall-Kation eines Übergangsmetalls der Gruppen 10, 11 oder 12, besonders bevorzugt der Gruppe 12 und am meisten bevorzugt ein $Zn^{2+}$-Kation

darstellen,

wobei der Verfahrensschritt iii) vor, während oder nach dem Verfahrensschritt ii) durchgeführt werden kann, vorzugsweise jedoch nach dem Verfahrensschritt ii) durchgeführt wird (was bedeutet, dass bereits oberflächennachvernetzte, wasserabsorbierende Polymergebilde mit der Verbindung der Struktur I in Kontakt gebracht werden).

Im Verfahrensschritt i) des erfindungsgemäßen Verfahrens werden zunächst wasserabsorbierende Polymergebilde bereitgestellt.

Als wasserabsorbierende Polymergebilde werden dabei im Verfahrensschritt i) vorzugsweise Polymere bereitgestellt, welche erhalten wurden durch ein Verfahren umfassend die Verfahrensschritte:

a) radikalische Polymerisation säuregruppen-tragender, ethylenisch ungesättigter, gegebenenfalls teilneutralisierter Monomere in Gegenwart eines Vernetzers unter Bildung eines Hydrogels;

b) gegebenenfalls Zerkleinern des Hydrogels;

c) zumindest teilweises Trocknen des gegebenenfalls zerkleinerten Hydrogels unter Erhalt wasserabsorbierender Polymergebilde;

d) gegebenenfalls Zermahlen des so erhaltenen absorbierenden Polymergebildes und absieben auf eine gewünschte Partikelgrößenfraktion;

e) gegebenenfalls weitere Oberflächenmodifizierungen der so erhaltenen wasserabsorbierenden Polymergebilde.

Die im Verfahrensschritt a) erfolgende radikalische Polymerisation erfolgt vorzugsweise in wässriger Lösung, wobei diese wässrige Lösung neben Wasser als Lösungsmittel vorzugsweise

($\alpha$1) die ethylenisch ungesättigten, säuregruppen-tragenden Monomeren oder deren Salze, wobei Acrylsäure als säuregruppentragendes Monomer besonders bevorzugt ist,
($\alpha$2) gegebenenfalls monoethylenisch ungesättigte, mit ($\alpha$1) copolymerisierbare Monomere,
($\alpha$3) den Vernetzer,
($\alpha$4) gegebenenfalls ein wasserlösliches Polymer, sowie
($\alpha$6) gegebenenfalls einen oder mehrere Hilfsmittel

beinhaltet.

Als monoethylenisch ungesättigte, mit ($\alpha$1) copolymerisierbare Monomere, wasserlösliche Polymere und Hilfsmittel sind wiederum diejenigen Verbindungen bevorzugt, die bereits eingangs im Zusammenhang mit den in der erfindungsgemäßen Zusammensetzung enthaltenen, wasserabsorbierenden Polymergebilden als mit ($\alpha$1) copolymerisierbare Monomere, als wasserlösliche Polymere bzw., als Hilfsmittel genannt wurden.

Aus den vorgenannten Monomeren, Comonomeren, Vernetzern, wasserlöslichen Polymeren und Hilfsstoffen lassen sich die wasserabsorbierende Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomeren ($\alpha$1) und ($\alpha$2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren ($\alpha$4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 43 40 706, DE 37 13 601, DE 28 40 010 und WO 96/05234 A1 beschrieben, deren entsprechende Offenbarung hiermit als Referenz eingeführt wird.

Die bei der Lösungspolymerisation oder der inversen Suspensions- und Emulsionspolymerisation im Verfahrensschritt a) erhaltenen Hydrogele werden im Verfahrensschritt c) zumindest teilweise getrocknet.

Insbesondere im Falle der Lösungspolymerisation ist es jedoch bevorzugt, dass die Hydrogele vor der Trocknung zunächst in einem zusätzlichen Verfahrensschritt b) zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 50 Gew.-%, vorzugsweise von 1 bis 25 Gew.-%, besonders bevorzugt 2 bis 10 Gew.-% erfolgt, wobei die Trocknungstemperaturen üblicherweise in einem Bereich von 100 bis 200°C liegen.

Die im Verfahrensschritt c) erhaltenen, zumindest teilweise getrockneten wasserabsorbierenden Polymergebilde können, insbesondere dann, wenn sie durch Lösungspolymerisation erhalten wurden, in einem weiteren Verfahrensschritt d) noch zermahlen und auf die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Im Anschluss an die Trocknung der Hydrogele und der gegebenenfalls erfolgten weiteren Konfektionierung der getrockneten wasserabsorbierenden Polymergebilde können diese in einem weiteren Verfahrenschritt e) im Oberflachenbereich modifiziert werden (ausgenommen von der im Verfahrensschritt e) durchgeführten Oberflächenmodifizierung ist die im nachfolgenden noch eingehender beschriebene Oberflächennachvernetzung gemäß Verfahrensschritt ii) und die Behandlung mit der Verbindung der Struktur I im Verfahrensschritt iii)).

Als bevorzugte Modifizierungsmaßnahme sei hier das in Kontakt bringen des Außenbereiches der Polymergebilde mit einer Verbindung enthaltend $Al^{3+}$-Ionen vor, während oder nach, vorzugsweise nach der Oberflächennachvernetzung gemäß Verfahrensschritt iii) zu nennen. Dabei ist es bevorzugt, dass die Verbindung enthaltend $Al^{3+}$-Ionen in einer Menge in einem Bereich von 0,01 bis 30 Gew. -%, besonders bevorzugt in einer Menge in einem Bereich von 0,05 bis 20 Gew. -% und darüber hinaus bevorzugt in einer Menge in einem Bereich von 0,1 bis 5 Gew. -%, jeweils bezogen auf das Gewicht der wasserabsorbierenden Polymergebilde, mit den wasserabsorbierenden Polymergebilden in Kontakt gebracht wird.

Das in Kontakt bringen des Außenbereiches der wasserabsorbierenden Polymergebilde mit der $Al^{3+}$-Ionen enthaltenden Verbindung erfolgt vorzugsweise dadurch, dass das Polymergebilde mit der Verbindung unter trockenen Bedingungen vermischt wird oder aber dadurch, dass die Polymergebilde mit einem Fluid $F_1$ umfassend ein Lösemittel, vorzugsweise Wasser, mit Wasser mischbare organische Lösemittel wie etwa Methanol oder Ethanol oder Mischungen aus mindestens zwei davon, sowie die $Al^{3+}$-Ionen enthaltende Verbindung in Kontakt gebracht werden, wobei das in Kontakt bringen vorzugsweise durch Besprühen der Polymerteilchen mit dem Fluid $F_1$ und Vermischen erfolgt. In diesem Zusammenhang ist es weiterhin bevorzugt, dass das in Kontakt bringen der Polymergebilde mit dem Fluid $F_1$ enthaltend die $Al^{3+}$-Ionen enthaltende Verbindung in einem zweistufigen Verfahren erfolgt. Dabei umfasst das zweistufige Verfahren einen ersten Mischvorgang, bei dem eine Vielzahl von Polymergebilden mit dem Fluid $F_1$ vermischt wird, und einem zweiten Mischvorgang, bei dem das Fluid $F_1$ im Inneren der Polymergebilde homogenisiert wird, wobei die Polymergebilde in dem ersten Mischvorgang mit einer Geschwindigkeit gemischt werden, dass die Bewegungsenergie der einzelnen Polymergebilde im Mittel größer ist als die Haftungsenergie zwischen den einzelnen Polymergebilden, und die Polymergebilde in dem zweiten Mischvorgang mit einer geringeren Geschwindigkeit als im ersten Mischvorgang durchmischt werden.

Durch die Behandlung der Polymergebilde mit dem Fluid $F_1$ beinhaltend die $Al^{3+}$-Ionen enthaltende Verbindung durch das vorstehend beschriebene, zweistufige Verfahren können Polymergebilde mit verbesserten Absorptionseigenschaften erhalten werden.

Vorzugsweise ist dabei die $Al^{3+}$-Ionen enthaltende Verbindung ohne Berücksichtigung von Kristallwasser in einer Menge in einem Bereich von 0,1 bis 50 Gew. - %, besonders bevorzugt in einer Menge in einem Bereich von 1 bis 30 Gew.-%, jeweils bezogen auf das Gesamtgewicht des Fluids $F_1$, in dem Fluid $F_1$ enthalten. Es ist weiterhin bevorzugt, dass das Fluid $F_1$ in einer Menge in einem Bereich von 0,01 bis 15 Gew.-%, besonders bevorzugt in einer Menge in einem Bereich von 0,05 bis 6 Gew.-%, jeweils bezogen auf das Gewicht der Polymergebilde, mit den Polymergebilden in Kontakt gebracht wird.

Bevorzugte $Al^{3+}$-Ionen enthaltenden Verbindungen sind $AlCl_3 \times 6H_2O$, $NaAl(SO_4)_2 \times 12\ H_2O$, $KAl(SO_4)_2 \times 12\ H_2O$, $Al_2(SO_4)_3 \times 14\text{-}18\ H_2O$ oder Aluminiumlactat.

Als weitere Oberflächenmodifizierungsmaßnahme sei an dieser Stelle das in Kontakt bringen der wasserabsorbierenden Polymergebilde mit anorganischen Partikeln, beispielsweise mit feinteiligem Siliziumdioxid, welches vorzugsweise in wässriger Suspension appliziert wird, oder mit Kieselsäuresol erwähnt.

Die vorstehend im Verfahrensschritt e) erfolgenden Modifizierungsmaßnahmen, insbesondere die Behandlung mit einer $Al^{3+}$-Ionen enthaltenden Verbindung, können grundsätzlich auch während der Durchführung der Verfahrensschritte ii) und iii), nach Durchführung der Verfahrensschritte ii) und iii), nach Durchführung des Verfahrensschrittes ii) aber vor der Durchführung des Verfahrensschrittes iii) oder aber nach Durchführung des Verfahrensschrittes iii) aber vor der Durchführung des Verfahrensschrittes ii) erfolgen.

Im Verfahrensschritt ii) erfolgt die Oberflächennachvernetzung des wasserabsorbierenden Polymergebildes, bei der die wasserabsorbierenden Polymergebilde zunächst mit einem Oberflächennachvernetzer in Kontakt gebracht und dann erhitzt werden. Dabei wird der Nachvernetzer insbesondere dann, wenn er unter den Nachvernetzungsbedingungen nicht flüssig ist, vorzugsweise in Form eines Fluids $F_2$ umfassend den Nachvernetzer sowie ein Lösungsmittel mit den Polymerteilchen in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid $F_2$ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht

des Fluids $F_2$, enthalten ist.

Das in Kontakt bringen des wasserabsorbierenden Polymergebildes mit dem Fluid $F_2$ beinhaltend den Nachvernetzer erfolgt im erfindungsgemäßen Verfahren vorzugsweise durch gutes Vermischen des Fluids $F_2$ mit dem wasserabsorbierenden Polymergebilde.

Geeignete Mischaggregate zum Aufbringen des Fluids $F_2$ sind zum Beispiel der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer). Auch können Mischvorrichtungen eingesetzt werden, in denen das Vermischen des Fluids $F_2$ mit dem wasserabsorbierenden Polymergebilde zumindest teilweise in einem sich drehenden Behälter erfolgt. Solche Mischvorrichtungen sind beispielweise von der Firma Lindor, Dordrecht, Niederlande erhältlich und werden beispielsweise unter den Produktbezeichnungen Lindor 70, Lindor 200, Lindor 500, Lindor 750, Lindor 1000, Lindor 1500, Lindor 2000, Lindor 2300, Lindor 4000, Lindor 7000, Lindor 8000, Lindor 12000, Lindor 14000 oder Lindor 25000 vertrieben.

Das wasserabsorbierende Polymergebilde wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew. -% an Lösungsmittel, vorzugsweise Wasser, in Kontakt gebracht und am allermeisten bevorzugt mit weniger als 3 Gew.-%, jeweils bezogen auf das Gewicht des wasserabsorbierenden Polymergebildes.

Bei wasserabsorbierenden Polymergebilden in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen, wasserabsorbierenden Polymergebilde mit dem Fluid $F_2$ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können oder aber polyvalente Metallkationen, die mittels elektrostatischer Wechselwirkung zwischen dem polyvalenten Metallkation und den funktionellen Gruppen eines Polymergebildes eine Vernetzung des Polymergebildes ermöglichen. Als Nachvernetzer sind im erfindungsgemäßen Verfahren diejenigen bevorzugt, die in WO 2004/037903 A2 als Vernetzer der Vernetzerklassen II und IV genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1, 3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die wasserabsorbierenden Polymergebilde mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der wasserabsorbierenden Polymergebilde infolge von Hitzeeinwirkung zerstört wird, begrenzt.

Im Verfahrensschritt iii) des erfindungsgemäßen Verfahrens werden die wasserabsorbierenden Polymergebilde mit der Verbindung der Struktur I in Kontakt gebracht, wobei es bevorzugt ist, dass die Reste $R^1$, $R^2$, $R^3$, $R^4$ und $R^5$ ein Wasserstoffatom darstellen, es sich bei der Verbindung der Struktur mithin also um Salicylsäure oder ein Metallsalz der Salicylsäure handelt, wobei der Einsatz von Salicylsäure, Natriumsalicylat und Zinksalicylat besonders bevorzugt und der Einsatz von Zinksalicylat am meisten bevorzugt ist.

Dabei ist es insbesondere bevorzugt, dass die Verbindung der Struktur I in einer Menge in einem Bereich von 0,001 bis 10 Gew.-%, besonders bevorzugt in einem Bereich von 0,05 bis 5 Gew.-% und am meisten bevorzugt in einer Menge in einem Bereich von 0,1 bis 3 Gew.-%, jeweils bezogen auf das Gesamtgewicht der wasserabsorbierenden Polymergebilde, mit den wasserabsorbierenden Polymergebilden in Kontakt gebracht wird.

Gemäß einer besonders bevorzugten Ausführungsform des erfindungsgemäßen Verfahrens wird die Verbindung der Struktur I in Form eines Pulvers, beinhaltend Partikel der Struktur I, eingesetzt, wobei es weiterhin bevorzugt ist, dass ein solches Pulver höchstens 25 Gew.-%, noch mehr bevorzugt höchstens 10 Gew.-%, darüber hinaus bevorzugt höchstens 5 Gew.-%, jeweils bezogen auf das Gesamtgewicht der Verbindung der Struktur I, am meisten bevorzugt jedoch keine vorzugsweise vereinzelten Partikel beinhaltet, die größer als 700 $\mu$m, besonders bevorzugt größer als 500 $\mu$m und am meisten bevorzugt größer als 300 $\mu$m im Durchmesser sind. Jedoch können in dem Pulver der Verbindung I durchaus auch Agglomerate aus mindestens zwei Partikeln enthalten sein, die größer als 700 $\mu$m, 500 $\mu$m bzw. 300

μm sind, wobei die Menge dieser Agglomerate durchaus auch größer als 25 Gew.-%, 10 Gew.-% bzw. 5 Gew.-% sein kann. Insbesondere kann es sich als vorteilhaft erweisen, dass das Pulver der Verbindung der Struktur I zu mindestens 50 Gew.-%, noch mehr bevorzugt zu mindestens 90 Gew.-% und am meisten bevorzugt ausschließlich auf Partikeln mit einem Durchmesser in einem Bereich von 50 bis 800 μm, besonders bevorzugt 50 bis 500 μm und am meisten bevorzugt 50 bis 350 μm basiert.

Das in Kontakt bringen der gegebenenfalls bereits oberflächennachvernetzten, wasserabsorbierenden Polymergebilde mit der Verbindung der Struktur I erfolgt vorzugsweise durch einfaches Vermischen der beiden Komponenten, wobei die Verbindung der Struktur I vorzugsweise in partikulärer Form mit dem wasserabsorbierenden Polymergebilde vermischt wird. Grundsätzlich denkbar ist jedoch auch, die Verbindung der Struktur I in Form einer Lösung oder einer Suspension, beispielsweise in Form einer wässrigen Lösung, mit dem wasserabsorbierenden Polymergebilde zu vermischen. Wie bereist vorstehend ausgeführt, hat sich jedoch insbesondere das Vermischen der Verbindung der Struktur I in partikulärer Form, insbesondere in Form eines Pulvers, mit den wasserabsorbierenden Polymergebilden als vorteilhaft erwiesen.

Geeignete Mischaggregate zum Vermischen der Verbindung der Struktur I und der wasserabsorbierenden Polymergebilde sind beispielsweise der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer). Denkbar ist auch hier der bereits im Zusammenhang mit der Oberflächennachvernetzung beschriebene Einsatz von Mischvorrichtungen, in denen das Vermischen der pulverförmigen Verbindung der Struktur I oder der Lösung beinhaltend Verbindung der Struktur I mit den wasserabsorbierenden Polymergebilden zumindest teilweise in einem sich drehenden Behälter erfolgt.

Nachdem die wasserabsorbierenden Polymergebilde mit der Verbindung der Struktur I in Kontakt gebracht worden sind, kann die so erhaltene wasserabsorbierende Zusammensetzung noch weiteren Modifizierungsmaßnahmen unterworfen werden. Denkbar ist hier insbesondere der Zusatz der eingangs im Zusammenhang mit der erfindungsgemäßen Zusammensetzung genannten Binder.

Einen Beitrag zur Lösung der eingangs genannten Aufgaben leistet auch eine wasserabsorbierende Zusammensetzung, welche durch das vorstehend beschriebene, erfindungsgemäße Verfahren erhältlich ist.

Es ist besonders bevorzugt, dass die eingangs beschriebene, erfindungsgemäße wasserabsorbierende Zusammensetzung und auch die durch das vorstehend beschriebene Verfahren erhältliche, erfindungsgemäße wasserabsorbierende Zusammensetzung mindestens eine der folgenden Eigenschaften aufweist:

(β1) eine nach ERT 441.2-02 bestimmte Retention von mindestens 20g/g, vorzugsweise mindestens 25g/g und besonders bevorzugt in einem Bereich von 25 bis 50 g/g;

(β2) eine (im Falle von Partikeln für die gesamte Partikelgrößenfraktion) nach ERT 442.2-02 bestimmte Absorption gegen einen Druck von 0,7 psi (50 g/cm$^2$) von mindestens 15 g/g, besonders bevorzugt von mindestens 20 g/g;

(β3) einen Neutralisationsgrad von höchstens 78 Mol-%, besonders bevorzugt von höchstens 70 Mol-% und mindestens 50 Mol-%;

(β4) eine nach ERT 450.2-02 bestimmte Rieselfähigkeit von mindestens 9 g/sec, besonders bevorzugt von mindestens 9,5 g/sec und am meisten bevorzugt von mindestens 10 g/sec;

(β5) eine gemäß der hierin beschriebenen Testmethode bestimmte Oberflächenspannung von mindestens 60 mN/m, besonders bevorzugt von mindestens 65 mN/m und am meisten bevorzugt von mindestens 70 mN/m.

Einen Beitrag zur Lösung der eingangs genannten Aufgaben leistet auch ein Verbund beinhaltend eine erfindungsgemäße wasserabsorbierende Zusammensetzung oder eine durch das erfindungsgemäße Verfahren erhältliche, wasserabsorbierende Zusammensetzung und ein Substrat.

Es ist dabei bevorzugt, dass die erfindungsgemäße, wasserabsorbierende Zusammensetzung und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich beinhaltet, welcher die erfindungsgemäße, wasserabsorbierende Zusammensetzung in einer Menge in einem Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm$^3$, vorzugsweise mindestens 0,1 cm$^3$ und am meisten bevorzugt mindestens 0,5 cm$^3$ aufweist.

In einer Ausgestaltung des erfindungsgemäßen Verbundes handelt es sich um einen flächenförmigen Verbund, wie er in der WO 02/056812 A1 als "*absorbent material*" beschrieben ist. Der Offenbarungsgehalt der WO 02/056812 A1, insbesondere hinsichtlich des genauen Aufbaus des Verbundes, des Flächengewichtes seiner Bestandteile sowie

seiner Dicke wird hiermit als Referenz eingeführt und stellt einen Teil der Offenbarung der vorliegenden Erfindung dar.

Einen weiteren Beitrag zur Lösung der eingangs genannten Aufgaben liefert ein Verfahren zur Herstellung eines Verbundes, wobei die erfindungsgemäße, wasserabsorbierende Zusammensetzung bzw. die durch das erfindungsgemäße Verfahren erhältliche, wasserabsorbierende Zusammensetzung und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung der eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Nach einem anderen Aspekt der vorliegenden Erfindung ist der Verbund als ein Hygieneartikelcore ausgebildet, das, jeweils bezogen auf das Hygieneartikelcore mindestens 30 Gew.- %, vorzugsweise mindestens 50 Gew.-% und darüber hinaus bevorzugt mindestens 70 Gew.- % der erfindungsgemäßen wasserabsorbierenden Zusammensetzung und mindestens 1 Gew.-%, vorzugsweise mindestens 5 Gew.-% und darüber hinaus bevorzugt mindestens 10 Gew.-% des Substrats und gegebenenfalls noch weitere übliche Hilfs- und/oder Haftmittel beinhaltet, wobei die Summe der Gewichtsprozente der einzelnen im Hygieneartikelcore beinhalteten Komponenten an 100 Gew.-% ergibt. Als Substrat im Zusammenhang mit dem Hygieneartikelcore sind besonders Materialien bevorzugt, die zur Fixierung der meist als Teilchen vorliegenden erfindungsgemäßen superabsorbierenden Zusammensetzung dienen. Hierbei kann es sich um Fasern oder Gewirke oder Gewebe sowie Netze handeln. Es ist weiterhin möglich, dass die beispielsweise als Pulver und damit teilchenförmig vorliegende wasserabsorbierende Zusammensetzung durch ein Haftmittel wie einen Klebstoff mit dem Substrat verbunden ist. Gleichfalls entspricht es einer Ausgestaltung, dass das Substrat so ausgestaltet ist, dass die wasserabsorbierende Zusammensetzung in Ausnehmung des Substrats aufgenommen wird. Übliche, ebenfalls in dem Hygieneartikelcore einarbeitbare Hilfsmittel sind beispielsweise Stoffe, wie kosmetische Stoffe, die die Hautverträglichkeit erhöhen, Desinfektionsmittel, antimikrobielle Substanzen und dergleichen.

In einem weiteren Aspekt betrifft die vorliegende Erfindung Hygieneartikel, beinhaltend eine flüssigkeitsdurchlässige Oberschicht, eine flüssigkeitsundurchlässige Unterschicht und einen zwischen der Oberschicht und der Unterschicht angeordneten erfindungsgemäßen Verbund. Als Hygieneartikel kommen sowohl Damenhygieneartikel, Erwachsenen-Inkontinenzprodukte als auch Windeln für Säuglinge, Babys und Kleinkinder in Betracht. Bevorzugt ist es, dass der Hygieneartikel ein vorstehend beschriebenes Hygieneartikelcore beinhaltet. Als flüssigkeitsdurchlässige Oberschicht kommen grundsätzlich alle dem Fachmann hierfür bekannten und geeignet erscheinenden Gewebe, Gelege und Gewirklagen, die meist aus Zellstoffen oder Zellstoffderivaten bestehen, die gegebenenfalls mit Kunststoffen wie Polypropylen oder Polyethylen bondiert sind, in Betracht. Auch als flüssigkeitsundurchlässige Unterschicht werden die in der Industrie dem Fachmann geläufigen meist ebenfalls aus einem Zellstoff oder Zellstoffderivat, - gelege, -gewirk, oder -gestrick bestehenden Vliese eingesetzt, wobei diese im Allgemeinen mit einer Kunststoffschicht, meist aus Polypropylen oder Polyethylen, abgedichtet sind.

Einen weiteren Beitrag zur Lösung der eingangs genannten Aufgaben liefern chemische Produkte beinhaltend die erfindungsgemäße, wasserabsorbierende Zusammensetzungen oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmende Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen, wasserabsorbierenden Zusammensetzung oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierenden Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Einen Beitrag zur Lösung der eingangs genannten Aufgaben leistet weiterhin die Verwendung einer Verbindung der Struktur I

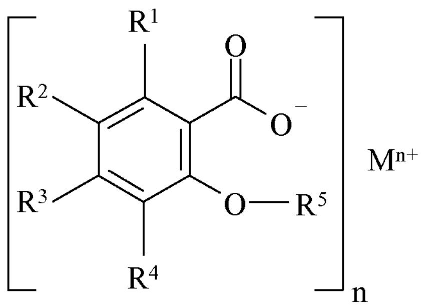

Struktur I

in der

- $R^1$, $R^2$, $R^3$ und $R^4$ gleich oder verschieden sein können und jeweils ein Wasserstoffatom, ein Halogen-Atom, beispielsweise ein Chlor-Atom oder ein Brom-Atom, eine $C_1$- bis $C_4$-Kohlenwasserstoff-Gruppe, vorzugsweise eine Methyl-Gruppe oder eine Ethylgruppe, besonders bevorzugt eine Methyl-Gruppe, oder eine Hydroxyl-Gruppe,

- $R^5$ ein Wasserstoffatom, eine $C_1$- bis $C_4$-Kohlenwasserstoff-Gruppe oder eine Acetyl-Gruppe, vorzugsweise ein Wasserstoffatom oder eine Acetyl-Gruppe und am meisten bevorzugt ein Wasserstoffatom,

- n eine ganze Zahl ausgewählt aus der Gruppe bestehend aus 1, 2 oder 3, besonders bevorzugt die Zahl 1 oder die Zahl 2 und am meisten bevorzugt die Zahl 2,

und

- $M^{n+}$ ein n-wertiges Metall-Kation oder ein $H^+$-Kation, vorzugsweise ein $H^+$-Kation, ein einwertiges Metall-Kation eines Alkalimetalls oder ein zweiwertiges Metall-Kation eines Erdalkali-Metalls oder eines Übergangsmetalls, besonders bevorzugt ein $H^+$-Kation, ein $Na^+$-Kation oder ein zweiwertiges Metall-Kationen eines Übergangsmetalls der Gruppen 10, 11 oder 12, besonders bevorzugt der Gruppe 12 und am meisten bevorzugt ein $Zn^{2+}$-Kation

darstellen,
insbesondere jedoch die Verwendung von Salicylsäure, Natriumsalicylat oder Zinksalicylat und am meisten bevorzugt die Verwendung von Zinksalicylat,
zur Verbesserung der geruchsbindenden Eigenschaften wasserabsorbierender Polymergebilde, wobei die wasserabsorbierenden Polymergebilde einen Innenbereich und einen den Innenbereich umgebenden Aussenbereich aufweisen, wobei der Aussenbereich einen höheren Vernetzungsgrad aufweist als der Innenbereich.

Die Erfindung wird nachfolgend anhand von Testmethoden und Figuren und Beispielen näher erläutert.

Es zeigt die Figur 1 den geruchsbindenden Effekt des erfindungsgemäßen Einsatzes von Zinksalicylat im Falle eines Superabsorbers mit einem Neutralisationsgrad von 55 Mol-% und im Falle eines Superabsorbers mit einem Neutralisationsgrad von 75 Mol-%.

TESTMETHODEN

Allgemein

Sofern nicht nachfolgend andere Testmethoden vorgegeben werden, wird auf die dem Fachmann allgemein bekannten und üblich erscheinenden Testmethoden zurückgegriffen, wobei insbesondere Testmethoden der EDANA (European Diaper and Nonwoven Association) Anwendung finden, die allgemein als "ERT-Methoden" angegeben werden.

Bestimmung der Oberflächenspannung

Bei der Bestimmung der Oberflächenspannung wird analysiert, in welchem Umfang eine bestimmte Menge an wasserabsorbierender Zusammensetzung, welche unter definierten Bedingungen in einer definierten Menge an 0,9 gew.-%iger NaCl-Lösung suspendiert worden ist, die Oberflächenspannung dieser wässrigen Lösung beeinflusst. Wenn die Oberflächenspannung durch den Geruchsbinder stark reduziert wird, so kann bei einem Einsatz der wasserabsorbierenden Zusammensetzung in Hygieneartikeln das Problem des "Rewets" auftreten. Ein "Rewet" führt dazu, dass Körperflüssigkeiten wie etwa Urin, welche bereits von dem Hygieneartikel absorbiert wurden, beispielsweise unter der Einwirkung eines äußeren Druckes wieder freigesetzt werden.

Alle zur Bestimmung der Oberflächenspannung eingesetzten Lösungen werden auf 23°C temperiert. Alle Geräte werden vor der Bestimmung ausreichend mit destilliertem Wasser gespült, mit Isopropanol entfettet und kurz über dem Bunsenbrenner mit blauer Flamme getrocknet.

Die Bestimmung der Oberflächenspannung erfolgt mittels der Ringmethode unter Einsatz des Interfacial Tensiometers K8 (Krüss GmbH, Hamburg). Dabei wird ein mit einem Waagesystem verbundener Platinring in die NaCl-Lösung eingetaucht und anschließend langsam wieder herausgezogen, wobei die Kraft bestimmt wird, welche erforderlich ist, um den Ring gegen die Grenzflächenspannung aus der Flüssigkeit herauszuziehen.

a) Konstruktion des Messgerätes

Zwischen 2 Klemmstücke ist ein Torsiondraht gespannt. Über ein Schneckengetriebe kann eine Torsion auf den Draht ausgeübt werden und das Tensiometer im hinteren Teil des Gerätes justiert werden. Das im vorderen Teil befindliche Getriebe ist fest mit einer Nonius-Skala verbunden. An dieser Skala kann der Wert der Grenzflächenspannung direkt in mN/m abgelesen werden. Der Waagebalken ist an dem Torsiondraht unter einem Winkel von 90° angeklemmt. Der Meßkörper wird mit seinem Stift in eine am unteren Ende des senkrechten Armes befindliche Ringaufnahme der Torsionwaage gesteckt. Der Meßkörper muss an seiner Unterseite vollständig plan sein, um ein gleichzeitiges Angreifen der Kräfte an allen Punkten zu ermöglichen. Durch Verdrehen des Torsiondrahtes wird der Meßkörper aus der Null-Lage ausgelenkt; der Ring wird gehoben oder gesenkt. Die vertikale Bewegung des Waagebalkens wird über ein Linsensytem auf einer Dunkelscheibe abgebildet.

b) Vorbereitung für die Messung

Nach Arretierung der Torsionwaage durch die Schraube, wird der Stift des Ringes in die Ringaufnahme der Waage gesteckt. Das Flüssigkeitsgefäß wird mittels Chrom-Schwefelsäure gereinigt, in destilliertem Wasser längere Zeit ausgekocht und vor Verwendung mit einem spitzflammigen Bunsenbrenner kurz ausgeflammt. Die zu untersuchende Flüssigkeit (25ml) wird in die erkaltete Glasschale geschüttet und in das Thermostatgefäß gesetzt. Zwischen diesem und dem Tisch sollte ein Abstand von 2cm bestehen. Dieser Abstand wird mittels der Mikrometerschraube eingestellt. Vor jeder Messung ist die Kreisteilung mit Schraube auf 0 zu stellen. Die Beleuchtung ist mit dem Schalter einzuschalten. Nach lösen der Arretierung sollte sich der Lichtzeiger auf der mittleren Linie der Dunkelscheibe einpendeln. Ist dies nicht der Fall, so wird die Nullstellung durch Drehung der an der Rückseite der am Kopfteil befindlichen Schraube nachjustiert. In diesem Zustand schwingt die Waage frei um ihre Null-Lage. Das Instrument ist nun meßbereit.

c) Messung der Oberflächenspannung

Der Thermostatentisch wird mit dem Handrad gehoben, bis der Ring vollständig mit Flüssigkeit bedeckt ist. Mit dem Handrad wird der Tisch vorsichtig gesenkt, bis der Lichtzeiger aufgrund der am Ring wirkenden Grenzflächenspannung aus der Null-Lage ausgelenkt wird. Durch Drehen der Schraube gegen den Uhrzeigersinn wird die Torsion des Bandes erhöht und ein Zug auf den Ring ausgeübt. Dabei verschiebt sich der auf der Dunkelscheibenskala zu beobachtende Lichtzeiger nach oben. Mit der Mikrometerschraube wird der Lichtzeiger durch Senken des Meßgefäßes in die Nullstellung zurückgebracht. Man wiederholt diese abwechselnde vorsichtige Erhöhung des Zuges und das darauffolgende Senken des Meßgefäßes so lange, bis der am Ring angreifende Zug die Grenzflächenspannungskräfte völlig überwindet und der Ring aus der Oberfläche nach oben abreißt. Am Nonius der Skala wird die Grenzflächenspannung in 0,1 mN/m abgelesen.

d) Eichung

Das Tensiometer K8 ist mit doppelt destilliertem Wasser bei 20°C = 72,6 mN/m geeicht. Sollte sich ein anderer Wert ergeben, so arbeitet man mit einer Korrekturzahl. Misst man für Wasser bei 20°C zum Beispiel nicht 72,6 mN/m, sondern 73,0 mN/m, so ist der Korrekturfaktor gleich dem Quotienten aus theoretischem und abgelesenem Wert.

$$72{,}6/73 = 0{,}994$$

Mit einem solchen Korreturfaktor sind die abgelesenen Werte zu multiplizieren.

e) Probenvorbereitung

150 g 0,9 %ige NaCl-Lösung werden in einem 250 ml-Becherglas vorgelegt und mit einem Magnetrührer (200 UpM) gerührt. 2 g des zu prüfenden Produktes werden langsam in die sich bildende Trombe der 0,9 %igen NaCl-Lösung gestreut. Nach beendetem Einstreuen wird die Lösung 3 Minuten gerührt. Anschließend lässt man die Lösung 15 Minuten stehen. 25 ml dieser Lösung werden mit einer Pipette in das Probengefäß des Tensiometers K 8 gefüllt und 5 Minuten später die Oberflächenspannung gemessen. Es werden 3 Messungen durchgeführt.

BEISPIELE

Beispiel 1a Bereitstellung eines wasserabsorbierenden Polymergebildes (Verfahrensschritt i))

Eine Monomerenlösung bestehend aus 300 g Acrylsäure, die zu 75 Mol-% mit Natronlauge neutralisiert wurde, 441 g Wasser, 0,9 g Polyethylenglykol-300-diacrylat und 1,35 g Allyloxypolyethylenglykolacrylsäureester, wurde durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g $H_2O$, 0,07 g 35%ge Wasserstoffperoxidlösung in 10 g $H_2O$ und 0,015 g Ascorbinsäure in 2 g $H_2O$) zugesetzt. Nachdem die Endtemperatur von ca. 100°C erreicht war, wurde das entstandene Gel gewölft, so dass ein Granulat mit etwa 1 bis 3 mm großen Teilchen erhalten wurde. Der Wassergehalt betrug ca. 50 %. Diese Teilchen wurden bei 150°C 120 Minuten lang getrocknet. Das getrocknete Polymerisat wurde grob zerstoßen, gemahlen und auf ein Pulver mit einer Partikelgröße von 150 bis 850 µm gesiebt (Pulver A).

Beispiel 2a Oberftächennachvernetzung eines wasserabsorbierenden Polymergebildes (Verfahrensschritt ii))

Zur Nachvernetzung wurden 100g des oben erhaltenen Pulvers A unter kräftigen Rühren mit einer Lösung aus 1 g 1,3-Dioxolan-2-on, 3 g Wasser vermischt und anschließend für 30 Minuten in einem Ofen, der auf 180°C temperiert war, erhitzt. Es wurde ein oberflächennachvemetztes, wasserabsorbierendes Polymergebilde (Pulver B) erhalten.

Beispiel 1b Bereitstellung eines wasserabsorbierenden Polymergebildes (Verfahrensschritt i))

Eine Monomerenlösung bestehend aus 300 g Acrylsäure, die zu 55 Mol-% mit Natronlauge neutralisiert wurde, 491 g Wasser, 0,9 g Polyethylenglykol-300-diacrylat und 1,35 g Allyloxypolyethylenglykolacrylsäureester wurde durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g $H_2O$, 0,07 g 35%ge Wasserstoffperoxidlösung in 10 g $H_2O$ und 0,015 g Ascorbinsäure in 2 g $H_2O$) zugesetzt. Nachdem die Endtemperatur von ca. 100°C erreicht war, wurde das entstandene Gel gewölft, so dass ein Granulat mit etwa 1 bis 3mm großen Teilchen erhalten wurde. Der Wassergehalt betrug ca. 50 %. Diese Teilchen wurden bei 150°C 120 Minuten lang getrocknet. Das getrocknete Polymerisat wurde grob zerstoßen, gemahlen und auf ein Pulver mit einer Partikelgröße von 150 bis 850 µm gesiebt (Pulver C).

Beispiel 2b Oberflächennachvernetzung eines wasserabsorbierenden Polymergebildes (Verfahrensschritt ii))

Zur Nachvernetzung wurden 100 g des oben erhaltenen Pulvers C unter kräftigen Rühren mit einer Lösung aus 1 g 1,3-Dioxolan-2-on, 3 g Wasser vermischt und anschließend für 30 Minuten in einem Ofen, der auf 180°C temperiert war, erhitzt. Es wurde ein oberflächennachvemetztes, wasserabsorbierendes Polymergebilde (Pulver D) erhalten.

Beispiel 3 In Kontakt bringen eines wasserabsorbierenden Polymergebildes mit Zinksalicylat (Verfahrensschritt iii))

Das im Beispiel 2a erhaltene, oberflächennachvernetzte, wasserabsorbierende Polymergebilde (Pulver B) wurde mit verschiedenen Mengen an Zinksalicylat-Pulver (erhalten von der Firma Vertellus Specialties Inc., Indianapolis, USA) vermischt (siehe die Angaben in der nachfolgenden Tabelle 1). Dabei wurden die erfindungsgemäßen Zusammensetzungen gemäß den Pulvern E und F erhalten:

Als weiterer Vergleich wurde das im Beispiel 2a erhaltene, oberflächennachvernetzte, wasserabsorbierende Polymergebilde (Pulver B) auch mit Zinkrizinoleat nachbehandelt (Pulver G).

Tabelle 1

| | Pulver B (Vergleich) | Pulver E | Pulver F | Pulver G (Vergleich) |
|---|---|---|---|---|
| Menge an zugegebener Zink-Verbindung [Gew.-%] | 0 | 1 | 3 | 1 |
| TB [g/g] | 34,0 | 34,1 | 32,2 | 31,3 |
| $AAP_{0,7\ psi}$ [g/g] | 20,5 | 20,7 | 20,6 | 18,7 |
| Rieselfähigkeit | 11 | 11 | 10,6 | 10,4 |
| Oberflächenspannung [mN/m] | 64,2 | 62,7 | 63,6 | 47,8 |

Darüber hinaus wurde auch das im Beispiel 2b erhaltene, oberflächennachvernetzte, wasserabsorbierende Polymergebilde (Pulver D) mit verschiedenen Mengen an Zinksalicylat-Pulver vermischt (siehe die Angaben in der nachfolgenden Tabelle 2). Dabei wurden die erfindungsgemäßen Zusammensetzungen gemäß den Pulvern H und I erhalten.

Als weiterer Vergleich wurde das im Beispiel 2b erhaltene, oberflächennachvernetzte, wasserabsorbierende Polymergebilde (Pulver D) auch mit Zinkrizinoleat (Pulver J) sowie mit 5 Gew.-% Tegosorb®A30-Lösung (enthält 15 Gew.-% Zinkrizinoleat; Pulver K) nachbehandelt.

Tabelle 2

| | Pulver D (Vergl.) | Pulver H | Pulver I | Pulver J (Vergl.) | Pulver K (Vergl.) |
|---|---|---|---|---|---|
| Menge an zugegebener Zink-Verbindung [Gew.-%] | 0 | 0,5 | 1 | 1 | 0,75[1)] |
| TB [g/g] | 30,3 | 30 | 30,5 | 29,6 | 29,0 |
| $AAP_{0,7\ psi}$ [g/g] | 21,1 | 19,9 | 20,4 | 20,6 | 18,9 |
| Rieselfähigkeit | 10,2 | n. B. | n. B. | 10,0 | 6,9 |
| Oberflächenspannung [mN/m] | 71,1 | 71 | 71,1 | 45,9 | 41,5 |
| [1)] es wurden 5 Gew.-% einer Tegosorb®A30-Lösung, welche 15 Gew.-% Zinkrizinoleat enthält, appliziert. | | | | | |

Den Ergebnissen der Tabellen 1 und 2 ist zu entnehmen, dass der erfindungsgemäße Zusatz des Zinksalicylates das Absorptionsverhalten der wasserabsorbierenden Polymergebilde nicht nachteilig beeinflusst.

<u>Beispiel 4</u> Bestimmung der geruchsbindenden Eigenschaften der erfindungsgemäßen Zusammensetzungen

Von den Pulvern B, D, E, G, H, I, J und K wurden die geruchsbindenden Eigenschaften bestimmt.

Die Bestimmung der geruchsbindenden Eigenschaften der erfindungsgemäßen Zusammensetzungen erfolgte über die Bestimmung der Ammoniakfreisetzung der wasserabsorbierenden Zusammensetzung.

Dazu wurden *Proteus mirabilis*-Zellen über Nacht bei 37°C auf Caso-Schrägagar angezogen. Pro Röhrchen wurde mit 5 ml künstlichem Urin abgeschwemmt. Die Bakteriensuspenion wurde mit künstlichem Urin und 1 g/l Fleischextrakt sowie 1 g/l Pepton auf einen Keimgehalt von ungefähr $10^5$ KBE/ml eingestellt (KBE = Koloniebildenden Einheiten).

Jeweils 1 g der wasserabsorbierenden Zusammensetzung wurden mit 33 ml des mit Bakterien versetzten, künstlichen Urins enthaltend 1 g/l Fleischextrakt und 1 g/l Pepton versetzt. Als Kontrolle wurde zum einen ein Ansatz ohne eine erfindungsgemäße Zusammensetzung sowie eine wasserabsorbierende Zusammensetzung ohne Zinksalicylat eingesetzt.

Die Gefäße wurden mit einem Gummistopfen, durch dessen Bohrung ein Dräger-Diffusionsröhrchen geführt wurde, verschlossen und bei 37°C in einem Brutschrank inkubiert Der freigesetzte Ammoniak wurde in ppm × h gemessen. Die Anfangskeimzahl (gemessen in KBE) des Urins wurde durch das Ausplattieren geeigneter Verdünnungen auf Nährmedienplatten bestimmt. Für die Ammoniakfreisetzung wurde der Mittelwert aus zwei Ansätzen errechnet.

Die geruchsbindenden Eigenschaften der erfindungsgemäßen Pulver E, H und I sowie der Vergleichsprodukte B, D, G, J und K sind der Figur 1 zu entnehmen. Dieser Figur kann entnommen werden, dass insbesondere die Kombination eines nur zu 55 Mol-% teilneutralisierten, wasserabsorbierenden Polymergebildes mit 1 Gew.-% Zinksalicylat zu einer wasserabsorbierenden Zusammensetzung führt, die auch nach 17 Stunden kein Ammoniak freisetzt. Darüber

hinaus kann der Figur 1 entnommen werden, dass eine Einsatz-Menge von 0,5 Gew.-% Zinksalicylat zu einer stärkeren Verzögerung der Ammoniak-Freisetzung führt als ein Einsatz von 1 Gew.-% Zinkrizinoleat. Auch führt eine Einsatz-Menge von 0,5 Gew.-% Zinksalicylat zu einer Verzögerung der Ammoniak-Freisetzung, die vergleichbar ist mit der Verzögerung bei einem Einsatz von 0,75 Gew.-% Zinkrizinoleat, welches in Form der Tegosorb A30-Dispersion appliziert wurde. Mithin kann Zinksalicylat im Vergleich zu dem aus dem Stand der Technik bekannten Zinkrizinoleat in geringeren Mengen eingesetzt werden.

**Patentansprüche**

1. Eine wasserabsorbierende Zusammensetzung beinhaltend wasserabsorbierende Polymergebilde, wobei diese einen Innenbereich und einen den Innenbereich umgebenden Außenbereich aufweisen, wobei der Außenbereich einen höheren Vernetzungsgrad aufweist als der Innenbereich, sowie eine Verbindung der Struktur I

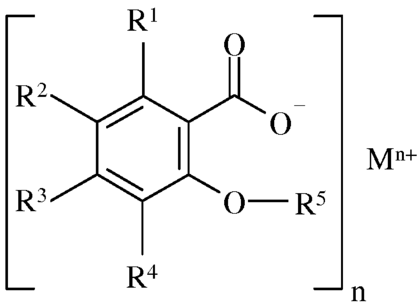

Struktur I

in der

- $R^1$, $R^2$, $R^3$ und $R^4$ gleich oder verschieden sein können und jeweils ein Wasserstoffatom, ein Halogen-Atom, eine $C_1$- bis $C_4$-Kohlenwasserstoff-Gruppe oder eine Hydroxyl-Gruppe,
- $R^5$ ein Wasserstoffatom, eine $C_1$- bis $C_4$-Kohlenwasserstoff-Gruppe oder eine Acetyl-Gruppe,
- n eine ganze Zahl ausgewählt aus der Gruppe bestehend aus 1, 2 oder 3, und
- $M^{n+}$ ein n-wertiges Metall-Kation oder ein $H^+$-Kation

darstellen.

2. Die wasserabsorbierende Zusammensetzung nach Anspruch 1, wobei $R^1$, $R^2$, $R^3$ und $R^4$ ein Wasserstoffatom, n gleich 2 und $M^{n+}$ ein zweiwertiges Metall-Kation eines Übergangsmetalls sind.

3. Die wasserabsorbierende Zusammensetzung nach Anspruch 2, wobei $M^{n+}$ das $Zn^{2+}$-Kation ist.

4. Die wasserabsorbierende Zusammensetzung nach einem der vorhergehenden Ansprüche, wobei die wasserabsorbierende Zusammensetzung erhältlich ist durch das in Kontakt bringen wasserabsorbierender Polymergebilde mit der Verbindung der Struktur I.

5. Die wasserabsorbierende Zusammensetzung nach Anspruch 4, wobei die Verbindung der Struktur I in einer Menge in einem Bereich von 0,01 bis 10 Gew.-%, bezogen auf das Gesamtgewicht der wasserabsorbierenden Polymergebilde, mit den wasserabsorbierende Polymergebilden in Kontakt gebracht wird.

6. Die wasserabsorbierende Zusammensetzung nach einem der vorhergehenden Ansprüche, wobei die wasserabsorbierenden Polymergebilde auf teilneutralisierten, vernetzten Polyacrylaten basieren.

7. Die wasserabsorbierende Zusammensetzung nach einem der vorhergehenden Ansprüche, wobei die Verbindung der Struktur I in Form eines Pulvers in der wasserabsorbierenden Zusammensetzung enthalten ist.

8. Die wasserabsorbierende Zusammensetzung nach Anspruch 7, wobei das Pulver der Verbindung der Struktur I über einen Binder auf der Oberfläche der wasserabsorbierenden Polymergebilde immobilisiert ist.

**9.** Ein Verfahren zur Herstellung einer wasserabsorbierenden Zusammensetzung, beinhaltend die Verfahrensschritte:

i) Bereitstellen eines wasserabsorbierenden Polymergebildes;
ii) Nachvernetzung des wasserabsorbierenden Polymergebildes;
ii) in Kontakt bringen des wasserabsorbierenden Polymergebildes mit einer Verbindung der Struktur I

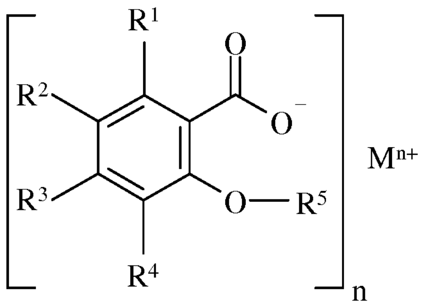

Struktur I

in der

- $R^1$, $R^2$, $R^3$ und $R^4$ gleich oder verschieden sein können und jeweils ein Wasserstoffatom, ein Halogen-Atom, eine $C_1$- bis $C_4$-Kohlenwasserstoff-Gruppe oder eine Hydroxyl-Gruppe,
- $R^5$ ein Wasserstoffatom, eine $C_1$- bis $C_4$-Kohlenwasserstoff-Gruppe oder eine Acetyl-Gruppe,
- n eine ganze Zahl ausgewählt aus der Gruppe bestehend aus 1, 2 oder 3, und
- $M^{n+}$ ein n-wertiges Metall-Kation oder ein $H^+$-Kation

darstellen,

wobei der Verfahrensschritt iii) vor, während oder nach dem Verfahrensschritt ii) durchgeführt werden kann.

**10.** Das Verfahren nach Anspruch 9, wobei $R^1$, $R^2$, $R^3$ und $R^4$ ein Wasserstoffatom und $M^{n+}$ ein zweiwertiges Metall-Kation eines Übergangsmetalls sind.

**11.** Das Verfahren nach Anspruch 10, wobei $M^{n+}$ das $Zn^{2+}$-Kation ist.

**12.** Das Verfahren nach einem der Ansprüche 9 bis 11, wobei die im Verfahrensschritt i) bereitgestellten, wasserabsorbierenden Polymergebilde auf teilneutralisierten, vernetzten Polyacrylaten basieren.

**13.** Das Verfahren nach einem der Ansprüche 9 bis 12, wobei die Verbindung der Struktur I in einer Menge in einem Bereich von 0,001 bis 10 Gew.-%, bezogen auf das Gesamtgewicht der wasserabsorbierenden Polymergebilde, eingesetzt wird.

**14.** Das Verfahren nach einem der Ansprüche 9 bis 13, wobei die Verbindung der Struktur I im Verfahrensschritt iii) in Form eines Pulvers eingesetzt wird.

**15.** Wasserabsorbierende Zusammensetzung, erhältlich durch ein Verfahren nach einem der Ansprüche 9 bis 14.

**16.** Wasserabsorbierende Zusammensetzung nach einem der Ansprüche 1 bis 8 oder 15, wobei die wasserabsorbierende Zusammensetzung mindestens eine der folgenden Eigenschaften aufweist:

($\beta$1) eine nach ERT 441.2-02 bestimmte Retention von mindestens 20g/g;
($\beta$2) eine (im Falle von Partikeln für die gesamte Partikelgrößenfraktion) nach ERT 442.2-02 bestimmte Absorption gegen einen Druck von 0,7 psi (50 g/cm$^2$) von mindestens 15 g/g;
($\beta$3) einen Neutralisationsgrad von höchstens 78 Mol-%;
($\beta$4) eine nach ERT 450.2-02 bestimmte Rieselfähigkeit von mindestens 9 g/g;
($\beta$5) eine gemäß der hierin beschriebenen Testmethode bestimmte Oberflächenspannung von mindestens 60 mN/m.

**17.** Ein Verbund, beinhaltend eine wasserabsorbierende Zusammensetzung nach einem der Ansprüche 1 bis 8, 15 oder 16 und ein Substrat.

**18.** Ein Verfahren zur Herstellung eines Verbundes, wobei eine wasserabsorbierende Zusammensetzung nach einem der Ansprüche 1 bis 8, 15 oder 16 und ein Substrat und gegebenenfalls ein Hilfsmittel miteinander in Kontakt gebracht werden.

**19.** Ein Verbund, erhältlich nach einem Verfahren gemäß Anspruch 18.

**20.** Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, beinhaltend die wasserabsorbierende Zusammensetzung nach einem der Ansprüche 1 bis 8, 15 oder 16 oder den Verbund nach Anspruch 17 oder 19.

**21.** Verwendung der wasserabsorbierenden Zusammensetzung nach einem der Ansprüche 1 bis 8, 15 oder 16 oder des Verbunds nach Anspruch 17 oder 19 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

**22.** Die Verwendung einer Verbindung der Struktur I

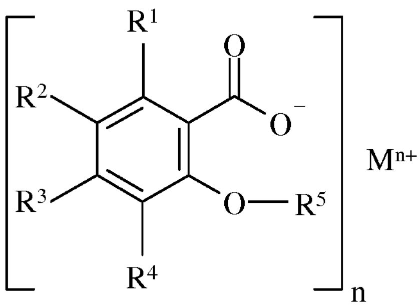

Struktur I

in der

- $R^1$, $R^2$, $R^3$ und $R^4$ gleich oder verschieden sein können und jeweils ein Wasserstoffatom, ein Halogen-Atom, eine $C_1$- bis $C_4$-Kohlenwasserstoff-Gruppe oder eine Hydroxyl-Gruppe,
- $R^5$ ein Wasserstoffatom, eine $C_1$- bis $C_4$-Kohlenwasserstoff-Gruppe oder eine Acetyl-Gruppe,
- n eine ganze Zahl ausgewählt aus der Gruppe bestehend aus 1, 2 oder 3, und
- $M^{n+}$ ein n-wertiges Metall-Kation oder ein $H^+$-Kation

darstellen,
zur Verbesserung der geruchsbindenden Eigenschaften wasserabsorbierender Polymergebilde, wobei diese einen Innenbereich und einen den Innenbereich umgebenden Aussenbereich aufweisen, wobei der Aussenbereich einen höheren Vernetzungsgrad aufweist als der Innenbereich..

## Claims

**1.** A water-absorbing composition comprising water-absorbing polymer structures, wherein said water-absorbing polymer structures have an inner region and an outer region surrounding the inner region, the outer region having a higher degree of crosslinking than the inner region, and a compound of the structure I

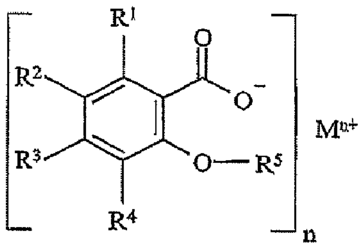

Structure I

in which

- $R^1$, $R^2$, $R^3$ and $R^4$ may be the same or different and are each a hydrogen atom, a halogen atom, a $C_1$ to $C_4$ hydrocarbon group or a hydroxyl group,
- $R^5$ is a hydrogen atom, a $C_1$ to $C_4$ hydrocarbon group or an acetyl group,
- n is an integer selected from the group consisting of 1, 2 and 3, and
- $M^{n+}$ is an n-valent metal cation or an $H^+$ cation.

**2.** The water-absorbing composition according to claim 1, wherein $R^1$, $R^2$, $R^3$ and $R^4$ are each a hydrogen atom, n is 2 and $M^{n+}$ is a divalent metal cation of a transition metal.

**3.** The water-absorbing composition according to claim 2, wherein $M^{n+}$ is the $Zn^{2+}$ cation.

**4.** The water-absorbing composition according to any one of the preceding claims, wherein the water-absorbing composition is obtainable by the contacting of water-absorbing polymer structures with the compound of the structure I.

**5.** The water-absorbing composition according to claim 4, wherein the compound of the structure I is contacted with the water-absorbing polymer structures in an amount within a range from 0.01 to 10% by weight, based on the total weight of the water-absorbing polymer structures.

**6.** The water-absorbing composition according to any one of the preceding claims, wherein the water-absorbing polymer structures are based on partly neutralized, crosslinked polyacrylates.

**7.** The water-absorbing composition according to any one of the preceding claims, wherein the compound of the structure I is present in the form of a powder in the water-absorbing composition.

**8.** The water-absorbing composition according to claim 7, wherein the powder of the compound of the structure I is immobilized by means of a binder on the surface of the water-absorbing polymer structures.

**9.** A process for producing a water-absorbing composition, comprising the process steps of:

i) providing a water-absorbing polymer structure;
ii) postcrosslinking the water-absorbing polymer structure;
iii) contacting the water-absorbing polymer structure with a compound of the structure I

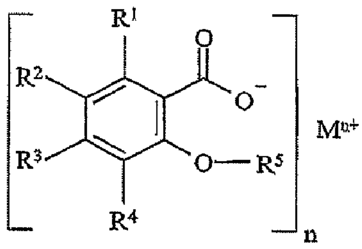

Structure I

in which

- $R^1$, $R^2$, $R^3$ and $R^4$ may be the same or different and are each a hydrogen atom, a halogen atom, a $C_1$ to $C_4$ hydrocarbon group or a hydroxyl group,
- $R^5$ is a hydrogen atom, a $C_1$ to $C_4$ hydrocarbon group or an acetyl group,
- n is an integer selected from the group consisting of 1, 2 and 3, and
- $M^{n+}$ is an n-valent metal cation or an $H^+$ cation,

wherein process step iii) can be performed before, during or after process step ii).

**10.** The process according to claim 9, wherein $R^1$, $R^2$, $R^3$ and $R^4$ are each a hydrogen atom and $M^{n+}$ is a divalent metal cation of a transition metal.

**11.** The process according to claim 10, wherein $M^{n+}$ is the $Zn^{2+}$ cation.

**12.** The process according to any one of claims 9 to 11, wherein the water-absorbing polymer structures provided in process step i) are based on partly neutralized, crosslinked polyacrylates.

**13.** The process according to any one of claims 9 to 12, wherein the compound of the structure I is used in an amount within a range from 0.001 to 10% by weight, based on the total weight of the water-absorbing polymer structures.

**14.** The process according to any one of claims 9 to 13, wherein the compound of the structure I is used in process step iii) in the form of a powder.

**15.** A water-absorbing composition obtainable by a process according to any one of claims 9 to 14.

**16.** The water-absorbing composition according to any one of claims 1 to 8 or 15, wherein the water-absorbing composition has at least one of the following properties:

($\beta$1) a retention determined to ERT 441.2-02 of at least 20 g/g;
($\beta$2) an absorption determined to ERT 442.2-02 (for the entire particle size fraction in the case of particles) against a pressure of 0.7 psi (50 g/cm$^2$) of at least 15 g/g;
($\beta$3) a degree of neutralization of at most 78 mol%;
($\beta$4) a free flow determined to ERT 450.2-02 of at least 9 g/g;
($\beta$5) a surface tension determined by the test method described herein of at least 60 mN/m.

**17.** A composite comprising a water-absorbing composition according to any one of claims 1 to 8, 15 and 16, and a substrate.

**18.** A process for producing a composite, wherein a water-absorbing composition according to any one of claims 1 to 8, 15 and 16, and a substrate and optionally an assistant are contacted with one another.

**19.** A composite obtainable by a process according to claim 18.

**20.** A foam, molding, fiber, foil, film, cable, sealing material, liquid-absorbing hygiene article, carrier for plant and fungus growth regulators, packaging material, soil additive or building material, comprising the water-absorbing composition according to any one of claims 1 to 8, 15 and 16, or the composite according to claim 17 or 19.

**21.** The use of the water-absorbing composition according to any one of claims 1 to 8, 15 and 16, or of the composite according to claim 17 or 19, in foams, moldings, fibers, foils, films, cables, sealing materials, liquid-absorbing hygiene articles, carriers for plant and fungus growth regulators, packaging materials, soil additives, for controlled release of active ingredients or in building materials.

**22.** The use of a compound of the structure I

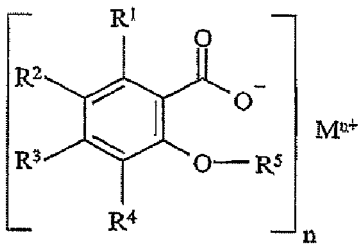

Structure I

in which

- $R^1$, $R^2$, $R^3$ and $R^4$ may be the same or different and are each a hydrogen atom, a halogen atom, a $C_1$ to $C_4$ hydrocarbon group or a hydroxyl group,
- $R^5$ is a hydrogen atom, a $C_1$ to $C_4$ hydrocarbon group or an acetyl group,
- n is an integer selected from the group consisting of 1, 2 and 3, and
- $M^{n+}$ is an n-valent metal cation or an $H^+$ cation, for improving the odor-binding properties of water-absorbing polymer structures, wherein said water-absorbing polymer structures have an inner region and an outer region surrounding the inner region, the outer region having a higher degree of crosslinking than the inner region.

**Revendications**

**1.** Composition absorbant l'eau, comportant des structures polymères absorbant l'eau, ces dernières présentant une zone interne et une zone externe entourant la zone interne, la zone externe présentant un plus haut degré de réticulation que la zone interne, ainsi qu'un composé de structure I

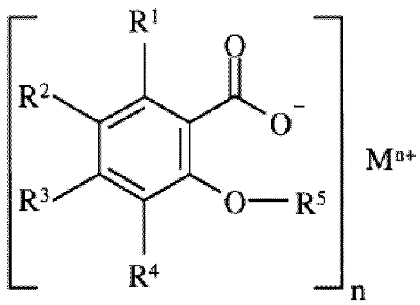

Structure I

dans laquelle

- $R^1$, $R^2$, $R^3$ et $R^4$ peuvent être identiques ou différents et représentent chacun un atome d'hydrogène, un atome d'halogène, un groupe hydrocarboné en $C_1$-$C_4$ ou un groupe hydroxy,
- $R^5$ représente un atome d'hydrogène, un groupe hydrocarboné en $C_1$-$C_4$ ou un groupe acétyle,
- n représente un nombre entier choisi dans le groupe constitué par 1, 2 et 3, et
- $M^{n+}$ représente un cation métallique n-valent ou un cation $H^+$.

**2.** Composition absorbant l'eau selon la revendication 1, dans laquelle $R^1$, $R^2$, $R^3$ et $R^4$ représentent un atome d'hydrogène, n est égal à 2 et $M^{n+}$ est un cation métallique divalent d'un métal de transition.

**3.** Composition absorbant l'eau selon la revendication 2, dans laquelle $M^{n+}$ est le cation $Zn^{2+}$.

**4.** Composition absorbant l'eau selon l'une quelconque des revendications précédentes, la composition absorbant l'eau pouvant être obtenue par la mise en contact de structures polymères absorbant l'eau avec le composé de structure I.

**5.** Composition absorbant l'eau selon la revendication 4, dans laquelle le composé de structure I est mis en contact, en une quantité dans une plage de 0,01 à 10 % en poids, par rapport au poids total des structures polymères absorbant l'eau, avec les structures polymères absorbant l'eau.

**6.** Composition absorbant l'eau selon l'une quelconque des revendications précédentes, dans laquelle les structures polymères absorbant l'eau sont à base de polyacrylates réticulés, partiellement neutralisés.

**7.** Composition absorbant l'eau selon l'une quelconque des revendications précédentes, dans laquelle le composé de structure I est contenu sous forme d'une poudre dans la composition absorbant l'eau.

**8.** Composition absorbant l'eau selon la revendication 7, dans laquelle la poudre du composé de structure I est immobilisée au moyen d'un agent de liaison sur la surface des structures polymères absorbant l'eau.

**9.** Procédé pour la préparation d'une composition absorbant l'eau, comprenant les étapes de procédé :

i) disposition d'une structure polymère absorbant l'eau ;
ii) post-réticulation de la structure polymère absorbant l'eau ;
iii) mise en contact de la structure polymère absorbant l'eau avec un composé de structure I

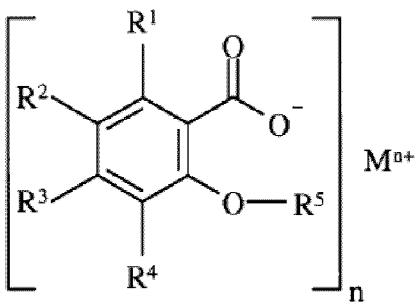

Structure I

dans laquelle

- $R^1$, $R^2$, $R^3$ et $R^4$ peuvent être identiques ou différents et représentent chacun un atome d'hydrogène, un atome d'halogène, un groupe hydrocarboné en $C_1$-$C_4$ ou un groupe hydroxy,
- $R^5$ représente un atome d'hydrogène, un groupe hydrocarboné en $C_1$-$C_4$ ou un groupe acétyle,
- n représente un nombre entier choisi dans le groupe constitué par 1, 2 et 3, et
- $M^{n+}$ représente un cation métallique n-valent ou un cation $H^+$,

l'étape iii) du procédé pouvant être effectuée avant, pendant ou après l'étape ii) du procédé.

**10.** Procédé selon la revendication 9, dans lequel $R^1$, $R^2$, $R^3$ et $R^4$ représentent un atome d'hydrogène et $M^{n+}$ est un cation métallique divalent d'un métal de transition.

**11.** Procédé selon la revendication 10, dans laquelle $M^{n+}$ est le cation $Zn^{2+}$.

**12.** Procédé selon l'une quelconque des revendications 9 à 11, dans lequel les structures polymères absorbant l'eau, disposées dans l'étape i) du procédé, sont à base de polyacrylates réticulés, partiellement neutralisés.

**13.** Procédé selon l'une quelconque des revendications 9 à 12, dans lequel le composé de structure I est utilisé en une quantité dans une plage de 0,001 à 10 % en poids, par rapport au poids total des structures polymères absorbant l'eau.

**14.** Procédé selon l'une quelconque des revendications 9 à 13, dans lequel on utilise dans l'étape iii) du procédé le composé de structure I sous forme d'une poudre.

**15.** Composition absorbant l'eau, pouvant être obtenue par un procédé selon l'une quelconque des revendications 9 à 14.

**16.** Composition absorbant l'eau selon l'une quelconque des revendications 1 à 8 ou 15, dans laquelle la composition absorbant l'eau présente au moins l'une des propriétés suivantes :

(β1) une rétention, déterminée selon ERT 441.2-02, d'au moins 20 g/g ;
(β2) une absorption déterminée (dans le cas de particules pour la fraction totale de tailles de particules) selon ERT 442.2-02 contre une pression de 0,7 psi (50 g/$cm^2$), d'au moins 15 g/g ;
(β3) un degré de neutralisation d'au maximum 78 % en moles ;
(β4) une coulabilité, déterminée selon ERT 450.2-02, d'au moins 9 g/g ;
(β5) une tension superficielle, déterminée selon la méthode d'essai décrite ici, d'au moins 60 mN/m.

**17.** Composite, comprenant une composition absorbant l'eau selon l'une quelconque des revendications 1 à 8, 15 ou 16, et un support.

**18.** Procédé pour la production d'un composite, dans lequel on met en contact entre eux une composition absorbant l'eau selon l'une quelconque des revendications 1 à 8, 15 ou 16 et un support et éventuellement un adjuvant.

**19.** Composite, pouvant être obtenu conformément à un procédé selon la revendication 18.

**20.** Mousses, corps moulés, fibres, feuilles, films, câbles, matériaux d'étanchéité, articles d'hygiène absorbant des liquides, supports pour agents régulateurs de la croissance de plantes et de champignons, matériaux d'emballage, additifs pour sols, ou matériaux de construction, qui comprennent la composition absorbant l'eau selon l'une quelconque des revendications 1 à 8, 15 ou 16 ou le composite selon la revendication 17 ou 19.

**21.** Utilisation de la composition absorbant l'eau selon l'une quelconque des revendications 1 à 8, 15 ou 16 ou du composite selon la revendication 17 ou 19 dans des mousses, corps moulés, fibres, feuilles, films, câbles, matériaux d'étanchéité, articles d'hygiène absorbant des liquides, supports pour agents régulateurs de la croissance de plantes et de champignons, matériaux d'emballage, additifs pour sols, pour la libération programmée de substances actives, ou dans des matériaux de construction.

**22.** Utilisation d'un composé de structure I

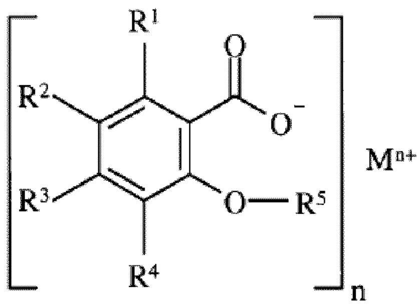

Structure I

dans laquelle

- $R^1$, $R^2$, $R^3$ et $R^4$ peuvent être identiques ou différents et représentent chacun un atome d'hydrogène, un atome d'halogène, un groupe hydrocarboné en $C_1$-$C_4$ ou un groupe hydroxy,
- $R^5$ représente un atome d'hydrogène, un groupe hydrocarboné en $C_1$-$C_4$ ou un groupe acétyle,
- n représente un nombre entier choisi dans le groupe constitué par 1, 2 et 3, et
- $M^{n+}$ représente un cation métallique n-valent ou un cation $H^+$,

pour l'amélioration des propriétés d'absorption des odeurs de structures polymères absorbant l'eau, ces dernières comportant une zone interne et une zone externe entourant la zone interne, la zone externe présentant un plus haut degré de réticulation que la zone interne.

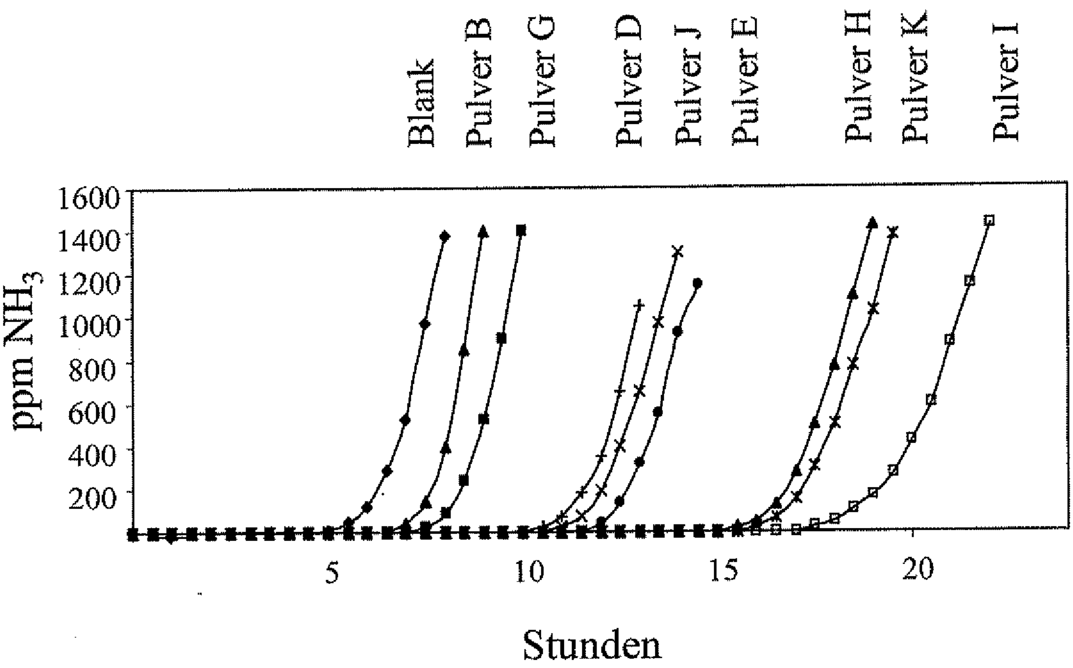
Blank
Pulver B
Pulver G
Pulver D
Pulver J
Pulver E
Pulver H
Pulver K
Pulver I
1600
1400
1200
1000
800
600
400
200
ppm $NH_3$
5
10
15
20
Stunden

Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS2612846 A
- DE 4020780 C1
- DE 19909653 A1
- DE 19909838 A1
- DE 19825486 A1
- DE 19939662 A1
- DE 10334271 A1
- DE 102005055497 A1
- WO 2005011860 A
- DE 19529348 A1
- WO 9934843 A1
- WO 2004037903 A2
- US 4286082 A
- DE 2706135
- US 4076663 A
- DE 3503458
- DE 4020780
- DE 4244548
- DE 4323001
- DE 4333056
- DE 4418818
- US 4340706 A
- DE 3713601
- DE 2840010
- WO 9605234 A1
- WO 02056812 A1


### In der Beschreibung aufgeführte Nicht-Patentliteratur


- Modern Superabsorbent Polymer Technology. Wiley-VCH, New York, 1998